(19) Europäisches Patentamt
European Patent Office
Office européen des brevets

(11) Publication number: **0 296 686 B1**

(12) **EUROPEAN PATENT SPECIFICATION**

(45) Date of publication of patent specification: **01.04.92**

(51) Int. Cl.$^5$: **C07C 271/06**, C07C 263/14

(21) Application number: **88201281.8**

(22) Date of filing: **21.06.88**

# (54) A process for the preparation of carbamates.

(30) Priority: **25.06.87 GB 8714919**

(43) Date of publication of application:
**28.12.88 Bulletin 88/52**

(45) Publication of the grant of the patent:
**01.04.92 Bulletin 92/14**

(84) Designated Contracting States:
**BE DE FR GB IT NL**

(56) References cited:
**EP-A- 0 086 281**
**EP-A- 0 169 650**
**EP-B- 0 097 592**
**GB-A- 1 469 222**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Stapersma, Johan**
**Badhuisweg 3**
**NL-1031 CM Amsterdam(NL)**
Inventor: **Steernberg, Koen**
**Badhuisweg 3**
**NL-1031 CM Amsterdam(NL)**

**EP 0 296 686 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of carbamates and/or derivatives thereof by reacting an organic compound containing at least one hydroxyl group with carbon monoxide and an organic nitrogenous compound with the aid of a catalytic system comprising palladium. Such processes have been described in United Kingdom patent specification 1,469,222 and European patent applications (publications) 86,281 and 169,650. Furthermore, it is known that the palladium catalyst is used together with nitrogen containing compounds as ligands. Examples of ligands are for example 2,2′-bipyridine and 1,10-phenanthroline, among many others.

Although the palladium forms an excellent homogeneous catalyst together with the ligand, like 2,2′-bipyridine and 1,10-phenanthroline, sometimes the fact that the ligand is not water-soluble may be a drawback.

Applicant has therefore sought for even more versatile ligands and has found that sulphonated 2,2′-bipyridine, sulphonated 1,10-phenanthroline or derivatives thereof, which are water-soluble, facilitate the working up of the products (carbamates) and facilitate the separation of the carbamates and the catalyst (palladium and ligand).

The invention relates to a process for the preparation of carbamates and/or derivatives thereof which comprises reacting an organic compound containing at least one hydroxyl group with carbon monoxide and an organic nitrogeneous compound in the presence of a catalytic system comprising palladium and/or a palladium compound and a water soluble sulphonated 2,2′-bipyridine, sulphonated 1,10-phenanthroline or sulphonated derivative of 2,2′-bipyridine or of 1,10-phenanthroline as ligand.

In European patent application 97,592 a process has been described, in which nitrobenzene is reduced to aniline with the aid of a rhodium, iridium, ruthenium or osmium catalyst complexed with a bidentate nitrogen compound e.g. a water-soluble sulphonated phenanthroline.

Suitable water-soluble sulphonated 1,10-phenanthrolines are the 3-sulphonated 1,10-phenanthroline; the 5-sulphonated 1,10-phenanthroline, the 5-sulphonated 4,7-diphenyl-1,10-phenanthroline and the 4,7-diphenyl-1,10-phenanthroline in which one or both of the phenyl groups contain a sulphonated group either in ortho-, meta- or para position. Sulphonated 1,10-phenanthroline derivatives may contain alkyl-, aryl-, alkylaryl-, alkoxy-, aryloxy-, alkylthio- or arylthio-groups, provided that these groups are not at the 2- and 9-position.

A preferred water-soluble sulphonated 1,10-phenanthroline has the chemical formula

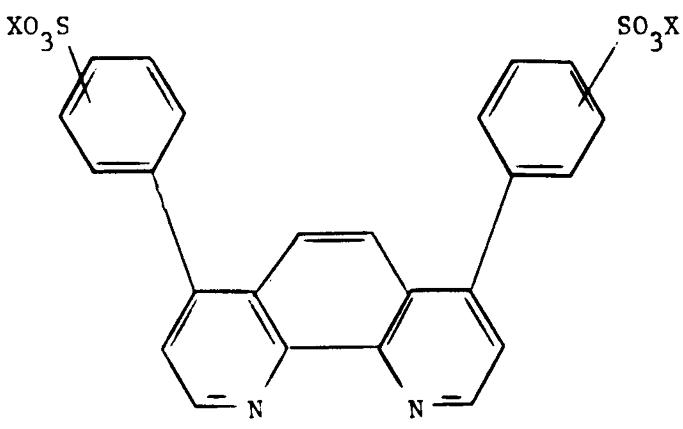

wherein X is hydrogen or a metal atom, preferably X is an alkali metal atom, more preferably sodium.

Another phenanthroline has the chemical formula

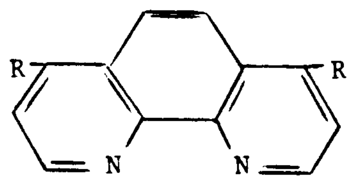

wherein R is a group $-CH_2-CH_2-$ 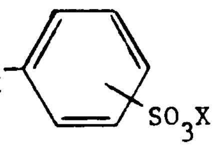

wherein X has the above-defined significance.

Another group of water-soluble ligands are sulphonated 2,2′-bipyridine and derivatives thereof. Examples are
5-sulphonated 2,2′-bipyridine,
4,4′-disulphonated 2,2′-bipyridine,
5,5′-disulphonated 2,2′-bipyridine,
4-sulphonated 2,2′-bipyridine.
Sulphonated 2,2′-bipyridine derivatives may contain alkyl-, aryl-, alkylaryl-, alkoxy-, aryloxy-, alkylthio- or arylthio-groups, provided that these groups are not at the 3,3′,6 and 6′ position.

A further water-soluble sulphonated 2,2′-bipyridine has the chemical formula

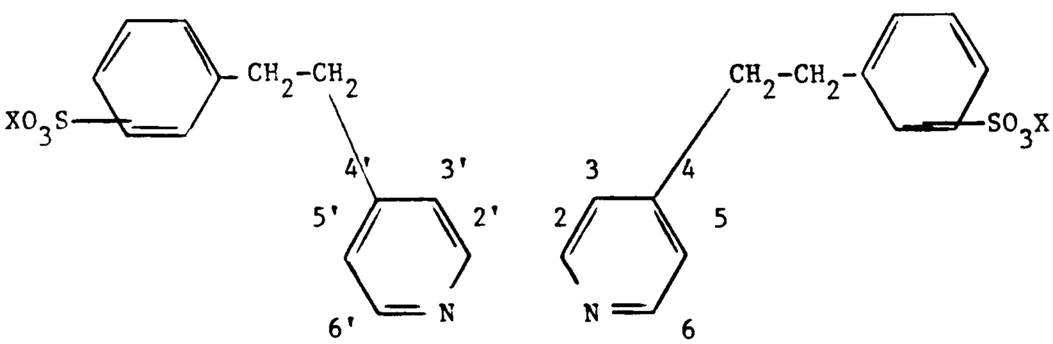

wherein X has the above-defined significance.

The sulphonated 1,10-phenanthrolines and 2,2′-bipyridines comprise the group-$SO_3X$, wherein X is H or a metal atom, preferably an alkali metal atom, more preferably the sodium atom.

The process according to the present invention is carried out in the presence of a catalyst based on palladium. Palladium can be used as such, deposited on an inert carrier such as carbon or alumina, or in the form of palladium compounds, especially palladium salts. Good results can also be obtained when a palladium compound is used which is substantially soluble in the prevailing reaction mixture. Examples of convenient palladium salts include palladium chloride, palladium bromide, palladium iodide, sodium tetrachloropalladate, potassium tetrachloropalladate, potassium tetra-iodopalladate, palladium acetate, palladium propionate, palladium isobutyrate, palladium acetylacetonate and similar palladium compounds. Preference is given to the use of palladium salts of organic acids, in particular palladium acetate.

It will be appreciated that the presence of a ligand together with palladium and/or a palladium compound is essential for obtaining the desired carbamates in high yields and with high selectivity.

The amount of palladium or palladium compound to be used in the process according to the present invention is conveniently between 0.001 %w and 10 %w, in particular between 0.005 %w and 3 %w, calculated on the amount of organic nitrogenous compound present in the reaction mixture. The use of rather small amounts of palladium compounds, e.g. less than 0.5 %w, calculated on organic nitrogeneous compound present in the reaction mixture is preferred.

The ligands should be used in such an amount that the ratio palladium (compound):ligand is conveniently between 0.05 and 50, preferably between 0.1 and 20.

The reaction according to the invention is preferably carried out in the presence of an acid which

causes an increase of the rate of conversion of the organic nitrogeneous compound, and in most cases also an increase of the selectivity to the desired carbamate.

Acids which may be used, are carboxylic acids having 1-6 carbon atoms, particularly acetic acid.

Although hydrogen halides, such as HCl, may be used, preference is given to acids which do not contain halogen anions. Hydrogen halides are less active as promoter and may give rise to corrosion. Examples of suitable acids which do not contain halogen anions are sulphuric, phosphoric, perchloric, fluoboric, fluosilicic and fluosulphonic acid. Sulphonic acids $RSO_3H$ in which R represents an optionally substituted hydrocarbon group are particularly preferred. The hydrocarbon group may be an alkyl, aryl, aralkyl or alkaryl group having 1-30, preferably 1-14 carbon atoms. The hydrocarbon group may be substituted with, for example, halogen atoms, particularly fluorine atoms. Preferred acids are p-toluene sulphonic acid and trifluoromethyl sulphonic acid. The acid $RSO_3H$ may also be an ionic exchange resin containing sulphonic acid groups such as, for example, Amberlite 252H, the group R being a polymeric hydrocarbon group, for example a polystyrene group substituted with sulphonic acid groups.

The amount of acid present in the reaction mixture preferably lies between 0.01 and 150, more preferably between 0.1 and 100, and most preferably between 1 and 50 equivalents per gram atom of palladium. Carboxylic acids such as acetic acid are preferably used in excess calculated on the molar amount of the ligand.

The carbamates are prepared from organic nitrogeneous compounds, i.e. compounds containing at least one non-cyclic group in which a nitrogen atom is directly attached to a single carbon atom and through a double bond to oxygen or another nitrogen atom such as organic nitro, nitroso, azo or azoxy compounds. Preference is given to aliphatic and, in particular, aromatic mono- and polynitro compounds.

Examples of aromatic nitro compounds include nitrobenzene, alkyl and alkoxy nitrobenzenes, aryl and aryloxy nitrobenzenes, dinitrobenzenes, alkyl and alkoxy, aryl and aryloxy dinitrobenzenes, such as 2,4-dinitrotoluene, 2,6-dinitrotoluene and 4,4′-dinitrodiphenylmethane, and polynitrobenzenes. Examples of aliphatic nitro compounds include nitromethane, nitroethane, 2,2-dimethylnitrobutane, nitrocyclopropane, 3-methyl nitrobutane, phenyl nitromethane, p-bromophenyl nitromethane, p-methoxyphenyl nitromethane, dinitroethane and dinitromethyl cyclohexane. Preferred organic nitro compounds are nitrobenzene, m-dinitrobenzene, nitrotoluene, 2,4-dinitrotoluene, 2,6-dinitroluene and 4,4′-dinitrodiphenylmethane.

Examples of nitroso compounds comprise aromatic nitroso compounds such as nitrosobenzene, nitrosotoluene, dinitrosobenzene and dinitrosotoluene as well as aliphatic nitroso compounds, such as nitrosobutane and nitrosocyclohexane.

Examples of azo compounds which can be used as starting materials in the process according to the present invention comprise azobenzene, nitroazobenzene, chloroazobenzene as well as alkyl- and aryl-substituted azobenzenes.

Examples of azoxy compounds which may be used as starting materials in the process according to the present invention include azoxybenzene, nitroazoxybenzene, chloroazoxybenzene as well as alkyl- and aryl-substituted azoxybenzenes.

Mixtures of two or more organic nitrogenous compounds belonging to the same or different classes as defined hereinbefore can also be applied, e.g. a mixture of two dinitrotoluenes or a mixture of a nitro compound and an azoxy compound.

It has further surprisingly been found that the preparation of carbamates from organic nitrogenous compounds containing a nitrogen-oxygen bond, in particular from aromatic nitro compounds, can even be improved further when the reaction is carried out in the presence of a primary or secondary amine or urea (derivative). The conversion of the organic nitrogenous compound can be increased whilst maintaining a high selectivity towards the desired carbamate, the primary or secondary amine or urea (derivatives) being co-converted into the desired carbamate.

As stated hereinabove, the process according to the present invention is carried out by reacting an organic nitrogenous compound with carbon monoxide and an organic compound containing at least one hydroxyl group. Mono- or polyhydric alcohols containing primary, secondary or tertiary hydroxyl groups as well as mixtures of such compounds can be used. The organic compounds containing at least one hydroxyl group can be represented by the general formula $R^1(OH)_m$, wherein m is an integer up to 4 and $R^1$ represents a substituted or unsubstituted alkyl, aryl, alkaryl or aralkyl group containing up to 20, preferably up to 6 carbon atoms.

Examples of compounds according to the general formula $R^1(OH)_m$, wherein m and $R^1$ are as defined hereinbefore comprise monohydric alcohols, such as methanol, ethanol, n-propanol, sec.-propanol, the butanols, amyl alcohol, hexyl alcohol, lauryl alcohol, cetyl alcohol, benzyl alcohol, chlorobenzyl alcohol, methoxy benzyl alcohol, methoxy ethanol, butoxy ethanol, cyclohexyl alcohol, phenol and the cresols. Examples of polyhydric alcohols comprise diols, e.g. ethylene glycol, diethylene glycol, propylene glycol,

dipropylene glycol and triols, such as glycerol, trimethylolpropane and the hexane triols. Ethers of the polyhydric compounds can also be used provided that they contain at least one free hydroxyl group in the molecule. Preference is given to the use of methanol, ethanol, propanol, isopropanol, butanol, sec.-butanol, isobutanol, ethylene glycol, glycerol, trimethylolpropane and phenol, in particular to methanol, ethanol and phenol.

The ratio of the organic nitrogenous compound to organic hydroxyl group-containing compound is not critical since any of those compounds can be used in excess to serve as solvent for the process according to the present invention. It has been found convenient to operate the process in the presence of a (large) excess of the organic hydroxyl group-containing compound but the use of an excess of the appropriate organic nitrogenous compound is by no means excluded. It is also possible to operate the process in the presence of an inert diluent such as an aliphatic or aromatic hydrocarbon, e.g. hexane, benzene or toluene, a halogenated hydrocarbon, such as a perfluoroalkane, or a ketone, ester or ether.

The process according to the present invention can be carried out conveniently at temperatures up to 300 °C. Preference is given to the use of temperatures in the range between 75 °C and 200 °C, in particular between 85 °C and 150 °C. The reaction is normally carried out at superatmospheric pressure; pressures of up to 500 bar can be applied. Preferably, the process is operated at rather low pressures. Good results can already be obtained using initial pressures between 30 and 70 bar. Mixtures of carbon monoxide and hydrogen containing up to about 20 %w of hydrogen can also be applied.

The process according to the present invention can be carried out batchwise, semi-continuously or continuously. The reaction time is, of course, related to the temperature and pressure adopted. In general, reaction times between 1 and 20 hours appears to be adequate.

After the reaction has taken place, the reaction mixture is treated with water and a water-immiscible organic solvent, the carbamate being dissolved in the latter while the catalyst components are dissolved in the water, and the water phase and organic phase are then separated. The catalyst components are then isolated from the aqueous solution and may be re-used in the reaction. The choice of water-immiscible organic solvent is dependant on the obtained carbamate. Preferred solvents are alkanes, benzene, toluene, and halogenated alkanes, e.g. methylene dichloride.

The carbamates produced according to the process according to the present invention can be used as such, for example as starting materials for agrochemicals, dyes, pharmaceuticals or polyurethanes, or may be converted into the corresponding isocyanates by methods known in the art. A suitable method therefore comprises heating the appropriate carbamate. The present invention therefore also relates to a process for the preparation of organic isocyanates by converting the carbamate and/or derivatives thereof which have been obtained by reacting an organic nitrogeneous compound, carbon monoxide and an organic compound containing at least one hydroxyl group in the presence of a catalytic system based on palladium and a water-soluble ligand, as hereinbefore mentioned.

## Example A

A 100 ml Hastelloy C autoclave was charged with nitrobenzene (25.9 mmol), dry methanol (20 ml), palladium acetate (0.03 mmol), 4,7-diphenyl-1,10-phenanthroline disulphonic acid disodium salt (0.3 mmol), and p-toluene sulphonic acid (0.06 mmol), and then pressurized with carbon monoxide (initial pressure 60 bar). Subsequently, the temperature in the autoclave was raised to 135 °C and the reaction mixture was kept at this temperature for 4 hours under magnetical stirring. After that period of time the reaction mixture was allowed to cool to ambient temperature and analyzed by gas-liquid chromatography (GLC). The conversion of nitrobenzene amounted to 100% and the selectivity to methyl carbanilate was 82%.

The obtained reaction mixture was concentrated by stripping of the methanol using a rotary evaporator at reduced pressure. The residue was dissolved in 200 ml dichloromethane and the solution extracted three times with 100 ml water to extract the catalyst. The combined aqueous extracts were washed once with 30 ml diethyl ether and subsequently, concentrated to a red-brown solid mass using a rotary evaporator at reduced pressure .

## Example B

The resulting red-brown solids were charged into the 100 ml autoclave. p-Toluene sulphonic acid (0.06 mmol), nitrobenzene (25.9 mmol), dry methanol (20 ml) were then charged into the autoclave.

Subsequently, the reaction was carried out exactly the same way as described above in the first run. The conversion of nitrobenzene amounted to 91% with a selectivity to methyl carbanilate of 60%.

## Claims

1. A process for the preparation of carbamates and/or derivatives thereof which comprises reacting an organic compound containing at least one hydroxyl group with carbon monoxide and an organic nitrogenous compound in the presence of a catalytic system comprising palladium and/or a palladium compound and a water soluble sulphonated 2,2′-bipyridine, sulphonated 1,10-phenanthroline or sulphonated derivative of 2,2′-bipyridine or of 1,10-phenanthroline as ligand.

2. A process as claimed in claim 1 in which the water-soluble ligand has the chemical formula

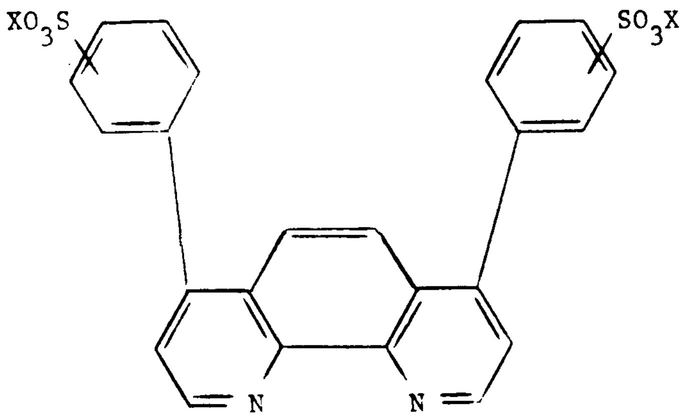

wherein X is hydrogen or a metal atom.

3. A process as claimed in claim 2, in which X is an alkali metal, preferably sodium.

4. A process as claimed in any one of the claims 1-3, in which the reaction is performed in the presence of an acid, as third catalyst component.

5. A process as claimed in claim 4, in which the acid is p-toluene sulphonic acid.

6. A process as claimed in any one of the claims 1-5, in which the reaction is performed at a temperature between 75 °C and 200 °C.

7. A process as claimed in any one of the claims 1-6, in which the reaction is performed at a pressure in the range of from 1 to 200 bar.

8. A process as claimed in any one of the claims 1-7, in which after the reaction has taken place, the reaction mixture is treated with water and a water-immiscible organic solvent, the carbamate being dissolved in the latter while the catalyst components are dissolved in the water, and the water phase and organic phase are then separated.

9. A process as claimed in claim 8, in which the water-immiscible organic solvent is methylene dichloride.

10. A process as claimed in claim 8 or 9, in which the catalyst components are isolated from the aqueous solution.

11. A process as claimed in any one of the claims 1-10 in which the catalyst components are re-used in the reaction.

12. A process for the preparation of organic isocyanates by converting a carbamate and/or derivatives thereof prepared by means of a process as claimed in any one of the claims 1-11, by methods known in the art, in particular by applying heat.

## Revendications

1. Procédé pour la préparation de carbamates et/ou de leurs dérivés, qui comporte la mise en réaction d'un composé organique contenant au moins un groupe hydroxyle avec le monoxyde de carbone et un composé organique azoté, en présence d'un système catalytique comportant du palladium et/ou un composé du palladium, et une 2,2'-bipyridine sulfonée, une 1,10-phénanthroline sulfonée, ou un dérivé sulfoné de la 2,2'-bipyridine ou de la 1,10-phénanthroline, solubles dans l'eau, comme ligand.

2. Procédé selon la revendication 1, où le ligand soluble dans l'eau a la formule chimique

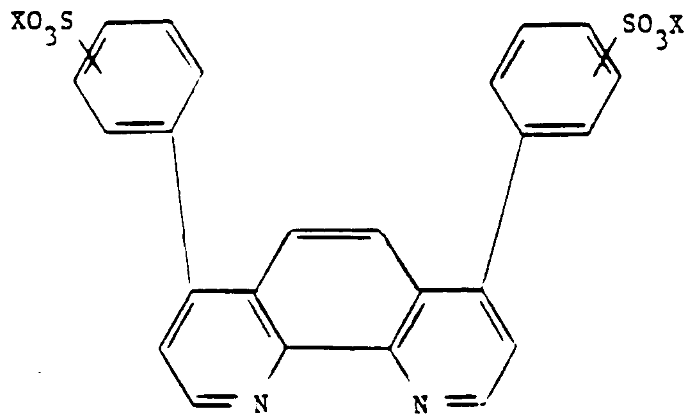

dans laquelle X est l'hydrogène ou un atome de métal.

3. Procédé selon la revendication 2, où X est un métal alcalin, de préférence le sodium.

4. Procédé selon l'une quelconque des revendications 1 à 3, où la réaction est mise en oeuvre en présence d'un acide, comme troisième composant catalytique.

5. Procédé selon la revendication 4, où l'acide est l' acide p-toluène sulfonique.

6. Procédé selon l'une quelconque des revendications 1 à 5, où la réaction est mise en oeuvre à une température comprise entre 75 ° C et 200 ° C.

7. Procédé selon l'une quelconque des revendications 1 à 6, où la réaction est mise en oeuvre à une pression dans le domaine de 1 à 200 bars.

8. Procédé selon l' une quelconque des revendications 1 à 7, où, après la mise en oeuvre de la réaction, le mélange réactionnel est traité avec de l'eau et un solvant organique non miscible à l'eau, le carbamate étant dissous dans ce dernier, alors que les composants catalytiques sont dissous dans l'eau, et la phase aqueuse et la phase organique sont alors séparées.

9. Procédé selon la revendication 8, où le solvant organique non miscible à l'eau est le dichlorure de méthylène.

10. procédé selon l'une quelconque des revendications 8 ou 9, où les composants catalytiques sont isolés de la solution aqueuse.

11. Procédé selon l'une quelconque des revendications 1 à 10, où les composants catalytiques sont réutilisés dans la réaction.

12. Procédé pour la préparation d'isocyanates organiques en transformant un carbamate et/ou ses dérivés, préparés au moyen d'un procédé tel que revendiqué dans l'une quelconque des revendications 1 à 11, selon des méthodes connues de l'art, en particulier par application de la chaleur.

**Patentansprüche**

1. Verfahren zur Herstellung von Carbamaten und/oder Derivaten davon, das umfaßt die Umsetzung einer organischen Verbindung, enthaltend mindestens eine Hydroxylgruppe, mit Kohlenmonoxid und einer organischen stickstoffhaltigen Verbindung in Gegenwart eines katalytischen Systems, umfassend Palladium und/oder eine Palladiumverbindung und ein wasserlösliches sulfoniertes 2,2'-Bipyridin, sulfoniertes 1,10-Phenanthrolin oder sulfoniertes Derivat von 2,2'-Bipyridin oder von 1,10-Phenanthrolin als Liganden.

2. Verfahren nach Anspruch 1, wobei der wasserlösliche Ligand die chemische Formel

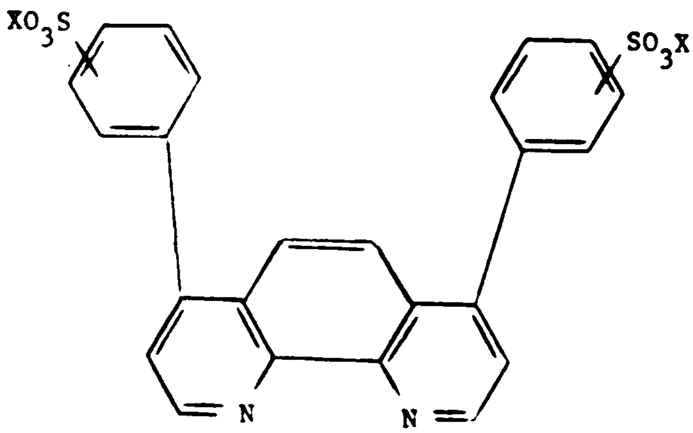

in der X Wasserstoff oder ein Metallatom ist, besitzt.

3. Verfahren nach Anspruch 2, wobei X ein Alkalimetall, vorzugsweise Natrium, ist.

4. Verfahren nach einem der Ansprüche 1 bis 3, wobei die Reaktion in Gegenwart einer Säure als dritte Katalysatorkomponente durchgeführt wird.

5. Verfahren nach Anspruch 4, wobei die Säure p-Toluolsulfonsäure ist.

6. Verfahren nach einem der Ansprüche 1 bis 5, wobei die Reaktion bei einer Temperatur zwischen 75 ° C und 200 ° C durchgeführt wird.

7. Verfahren nach einem der Ansprüche 1 bis 6, wobei die Reaktion bei einem Druck im Bereich von 1 bis 200 bar durchgeführt wird.

8. Verfahren nach einem der Ansprüche 1 bis 7, wobei nach Ablauf der Reaktion das Reaktionsgemisch mit Wasser und einem mit Wasser nicht mischbaren organischen Lösungsmittel behandelt wird, wobei das Carbamat sich in dem Letzteren löst, während die Katalysatorkomponenten in dem Wasser gelöst werden, und die wäßrige Phase und die organische Phase anschließend getrennt werden.

9. Verfahren nach Anspruch 8, wobei das mit Wasser nicht mischbare organische Lösungsmittel Methylendichlorid ist.

10. Verfahren nach Anspruch 8 oder 9, wobei die Katalysatorkomponenten aus der wäßrigen Lösung isoliert werden.

11. Verfahren nach einem der Ansprüche 1 bis 10, wobei die Katalysatorkomponenten wieder für die Reaktion verwendet werden.

12. Verfahren zur Herstellung von organischen Isocyanaten durch Umwandlung eines Carbamats und/oder von Derivaten davon, hergestellt mit Hilfe eines Verfahrens nach einem der Ansprüche 1 bis 11, nach bekannten Verfahren, insbesondere durch Anwendung von Wärme.